# EUROPEAN PATENT APPLICATION

(11) **EP 4 464 692 A1**
(43) Date of publication of application: **20.11.2024**
(21) Application number: 23740019.7
(22) Date of filing: 10.01.2023
(51) Int. Cl.: C07D 207/34, C07D 453/04

(54) **METHOD FOR PREPARING PYRROLE COMPOUND AND INTERMEDIATE THEREOF**

(30) Priority: 14.01.2022 CN 202210041776
(71) Applicant: Sunshine Lake Pharma Co., Ltd., Dongguan, Guangdong 523000 (CN)
(72) Inventor: YANG, Chuanwen, Dongguan, Guangdong 523871 (CN); WANG, Jiancheng, Dongguan, Guangdong 523871 (CN); ZHANG, Yingxun, Dongguan, Guangdong 523871 (CN); DING, Xiaohong, Dongguan, Guangdong 523871 (CN)
(74) Representative: Hoffmann Eitle
(86) International application number: PCT/CN2023/071604
(87) International publication number: WO 2023/134677

(57) **Abstract**

The present application relates to a method for preparing a pyrrole compound and an intermediate thereof. The pyrrole compound and/or the intermediate thereof can be used for preparing a pyrrole amide compound serving as a mineralocorticoid receptor antagonist. Specifically, the provided preparation method has a mild preparation condition, is simple in operation, is safe and controllable, has high total yield, and is suitable for industrial production.

## Description

### FIELD OF THE INVENTION

The present invention refers to a chemical medicine field. Specifically, the invention relates to processes for preparing a pyrrole compound, and also relates to important intermediates and the preparation methods thereof. The pyrrole compound and/or intermediates thereof of the present invention may be used to prepare pyrrole amide compounds used as mineralocorticoid receptor antagonists.

### BACKGROUND ART

International application WO2021078135A1 discloses a class of pyrrole amide compounds and uses thereof, which can be used as mineralocorticoid receptor antagonists and have potential therapeutic efficacy for diseases such as hyperaldosteronism, hypertension, chronic heart failure, sequelae of myocardial infarction, cirrhosis, non-alcoholic steatohepatitis, chronic kidney disease, diabetic nephropathy, renal failure, fibrosis and/or stroke. Specifically, the international application discloses the compound shown in Formula (A) and the preparation method as follows: a racemic compound of Formula (A) is obtained by substitution, hydrolysis, acyl chlorination, condensation and deprotection reactions of *N-*(3-fluoro-4-(methylsulfonyl)phenyl)-1-(2-hydroxyethyl)-4-methyl-5-(2-(trifluoromethyl)phenyl)-1*H*-pyrrole-3-carboxamide as raw material, and then a compound of Formula (A) is obtained by chiral column resolution. The method involves a chiral resolution method for separating stereoisomers, and the separation method has high requirements for instruments, low yield, high production cost and is not suitable for industrial production; on the whole, the preparation method is not suitable for industrial scale-up production due to its high production cost, poor atomic economy, large environmental pollution, and harsh reaction conditions.

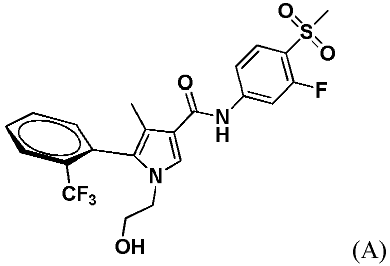

### SUMMARY

The present invention provides intermediates that can be used for the preparation of a compound of Formula (A) and preparation methods thereof; wherein the intermediates comprise pyrrole compounds shown in each general formula and/or each structural formula of the present invention.

The present invention relates to a method for preparing pyrrole compounds, and also to important intermediates in the method and preparation methods thereof. The preparation methods of the present invention have mild conditions, simple operation, safety and controllability, high overall yield and suitable for industrial production.

The pyrrole compounds prepared by the methods described in the present invention can be used to prepare the compound shown in Formula (A).

In one aspect, the present invention provides a method for preparing compound of Formula (A), 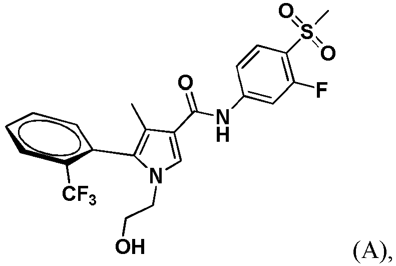 wherein, the method comprises:
2a) a compound of Formula (V) is hydrolyzed to obtain a compound of Formula (VI); 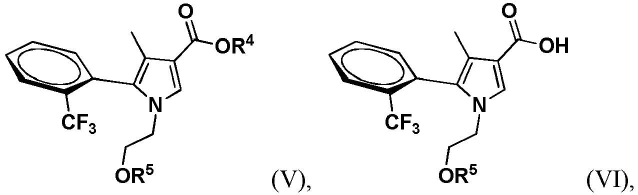
2b) the compound of Formula (VI) reacts with dichlorosulfoxide, oxalyl chloride or N,N'-carbonyl diimidazole (CDI) to obtain a compound of Formula (VII), 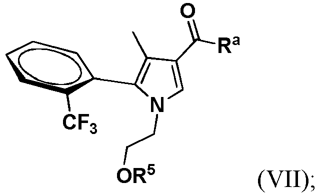
2c) the compound of Formula (VII) undergoes ammonolysis reaction to obtained a compound of Formula (VIII), 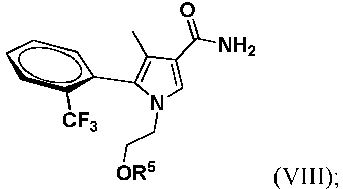
2d) the compound of Formula (VIII) reacts with a compound of Formula (IX) to obtain a compound of Formula (X), 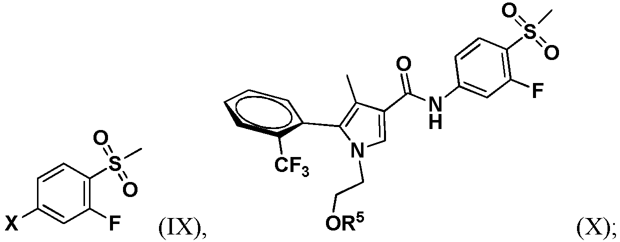
2e) the compound of Formula (X) is deprotected to obtain the compound of Formula (A);
wherein, X is Br or I; R^{a} is Cl or 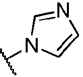 ; R⁴ and R⁵ are as defined herein.

In some embodiments, the deprotection reaction of step 2e) of the present invention is carried out in a hydrogen atmosphere in the presence of palladium carbon.

In some embodiments, the deprotection reaction of step 2e) of the present invention may be carried out under acidic conditions or in the presence of acidic substances.

In some embodiments, the deprotection reaction of step 2e) of the present invention is carried out under the condition that Lewis acid is present. In some other embodiments, the Lewis acid is zinc chloride (ZnCl₂), titanium tetrachloride or aluminum chloride, etc.

In other embodiments, the acidic condition means the condition in which the acid exists, and the acid is a suitable acid applicable for the deprotection reaction, including but not limited to hydrochloric acid, acetic acid, trifluoroacetic acid, etc., and preferably, the acid is hydrochloric acid, acetic acid, trifluoroacetic acid, or a solution thereof.

In still other embodiments, in step 2e) of the present invention, the amount of the acid is 0.17 mL/g to 0.34 mL/g with respect to the mass of the compound shown in Formula (X). In still other embodiments, the acid of the present invention is hydrochloric acid, and the amount of hydrochloric acid is 0.17 mL/g to 0.34 mL/g. In still other embodiments, hydrochloric acid refers to an aqueous solution of hydrogen chloride. Specifically, the concentration of hydrochloric acid is greater than 1 mol/L; preferably the concentration of hydrochloric acid is 1 mol/L to 12 mol/L; and preferably, the concentration of hydrochloric acid is 3 mol/L to 12 mol/L.

In some embodiments, the reaction temperature of the deprotection reaction in the step 2e) of the present invention is 50 °C-75 °C; and preferably, the reaction temperature is 55 °C-70 °C.

In some embodiments, the deprotection reaction of step 2e) of the present invention can be carried out under the condition of atmospheric pressure (0.1 MPa), and can also be carried out under the condition of greater than atmospheric pressure. In other embodiments, the pressure of the deprotection reaction of step 2e) of the present invention is 0.1 MPa to 3.8 MPa. In still other embodiments, the pressure of the deprotection reaction of step 2e) of the present invention is 0.8 MPa to 3.8 MPa.

In some embodiments, the deprotection reaction of step 2e) of the present invention is carried out in an alcohol solvent, and the alcohol solvent is methanol, ethanol, isopropanol, tert-butanol or tert-amyl alcohol. Preferably, the amount of the alcohol solvent may be 10 mL/g, based on the mass of the compound shown in Formula (X).

In some embodiments, the crude product obtained after the deprotection reaction of step 2e) of the present invention is purified by recrystallization to obtain a pure compound shown in Formula (I). In other embodiments, the recrystallization of the present invention is carried out in a mixed solvent of ethanol and water. In still other embodiments, the volume ratio of ethanol to water may be any appropriate ratio, including but not limited to 1:2-1:15. In other embodiments, the recrystallization of the present invention may be carried out in a mixed solvent of isopropyl acetate and toluene. In still other embodiments, the volume ratio of isopropyl acetate to toluene may be any appropriate ratio, including but not limited to 1:2-1:15; and specifically, the volume ratio of isopropyl acetate to toluene may be 1:5.

The purification of the compound shown in Formula (A) of the present invention can be achieved by recrystallization several times, each time under the same or different conditions. When necessary, the compound shown in Formula (A) of the present invention may be further purified by removing impurities through other purification methods commonly used in the art, for example, by removing residual palladium by using palladium-removing silica gel and/or activated carbon. The recrystallization can be carried out by first dissolving the crude product in a benign solvent, stirring until dissolved, and then adding a poor solvent or adding the solution of the crude product to a poor solvent, stirring to precipitate the solid. The dissolving process can be carried out under normal temperature conditions, or can also be carried out under the conditions of heating. Preferably, after dissolving the crude product with a benign solvent, the impurities can be removed by means such as using palladium-removing silica gel and/or activated carbon, and then the solution of the crude product can be added to a poor solvent to precipitate the solids. Preferably, in the above recrystallization, the benign solvent can be ethanol or isopropyl acetate, and the poor solvent can be water or toluene. In some embodiments, the benign solvent is ethanol and the poor solvent is water, preferably, the total volume ratio of ethanol to water may be about 1:1.5 to about 1:15, specifically, it may be about 1:1.8 to about 1:15. In other embodiments, the benign solvent is isopropyl acetate and the poor solvent is toluene; preferably, the total volume ratio of isopropyl acetate to toluene may be about 1:2 to about 1:15, specifically, may be about 1:4 to about 1:5.

In some embodiments, R⁵ is a suitable hydroxyl protecting group, including but not limited to, alkyl, alkenyl, alkoxyalkyl, alkylthioalkyl, silicoalkyl, substituted or unsubstituted benzyl, etc.; preferably, R⁵ is C₁₋₄ alkyl or benzyl, wherein the C₁₋₄ alkyl and benzyl are optionally substituted with 1, 2, 3 or 4 substituents independently selected from halo, C₁₋₄ alkyl, C₁₋₄ haloalkyl, C₁₋₄ alkoxy and C₁₋₄ haloalkoxy, respectively; more preferably, R⁵ is benzyl, p-methylbenzyl or p-methoxybenzyl.

In some embodiments, the reaction of step 2d) is carried out in the presence of base 1. Preferably, the base 1 is potassium carbonate, cesium carbonate, sodium carbonate, potassium *tert*-butoxide or potassium phosphate.

In other embodiments, the amount of base 1 is multiple times the molar amount of the compound shown in Formula (VIII), including but not limited to 1.2 times to 6.0 times; and in still other embodiments, the amount of base 1 is 1.5 times to 3.0 times the molar amount of the compound shown in Formula (VIII), preferably 1.5 to 2.0 times.

In some embodiments, the reaction of step 2d) is carried out in the presence of a catalyst, which is a metal catalyst.

In other embodiments, the catalyst is a palladium catalyst. Preferably, the palladium catalyst is Pd₂(dba)₃, Pd(dppf)Cl₂·CH₂Cl₂ or Pd(OAc)₂.

In other embodiments, the amount of the catalyst is 0.5%~5%, preferably 1%~3%, more preferably 1.5%~3%, of the molar amount of the compound shown in Formula (VIII).

In some embodiments, the reaction of step 2d) is carried out in the presence of a ligand. In other embodiments, the ligand is DMEDA, Xantphos, X-phos or S-phos.

In other embodiments, the amount of the ligand is 0.5%~5%, preferably 1%~3%, more preferably 1.5%~3%, of the molar amount of the compound shown in Formula (VIII).

In some embodiments, the reaction of step 2d) is carried out in the presence of a catalyst and a ligand. In other embodiments, the molar ratio of the catalyst to the ligand is 1: 1.

In other embodiments, the catalyst is Pd(OAc)₂ and the ligand is Xantphos, S-phos or X-phos, or the catalyst is Pd₂(dba)₃ and the ligand is Xantphos, S-phos or X-phos; or the catalyst is Pd(dppf)Cl₂·CH₂Cl₂ and the ligand is Xantphos, S-phos or X-phos.

In other embodiments, the catalyst is CuI, the ligand is DMEDA, and the base 1 is potassium carbonate.

In some embodiments, the reaction of step 2d) is carried out in solvent 1, which is toluene, dioxane (Dioxane), *tert*-butanol (t-BuOH), *tert*-amyl alcohol (*t*-AmOH), dimethyl ether (DME), cyclopentyl methyl ether (CPME), *N,N-*dimethylacetamide (DMAC), *N,N*-dimethylformamide (DMF), water or any combination thereof.

In some embodiments, the reaction temperature of step 2d) is 55 °C-110 °C, more preferably the reaction temperature is 55 °C-100 °C, and more preferably the reaction temperature is 55 °C-83 °C.

In some embodiments, the crude product of the compound of Formula (X) prepared according to the method described in the present invention can be purified by a purification method. The purification method includes, but is not limited to, recrystallization, using palladium-removing silica gel and/or activated carbon to remove impurities, etc.; and if necessary, multiple recrystallizations may be performed. The recrystallization can be carried out by first dissolving the crude product in a benign solvent, stirring until dissolved, then adding a poor solvent or adding the solution of the crude product to a poor solvent, stirring to precipitate a solid. The dissolution process can be carried out under a normal temperature condition, and can also be carried out under the condition of heating. Preferably, after dissolving the crude product with a benign solvent, the impurities can be removed by means such as using palladium-removing silica gel and/or activated carbon, and then the solution of the crude product can be added to a poor solvent to precipitate a solid. In some embodiments, the benign solvent is ethanol and the poor solvent is water, preferably, the total volume ratio of ethanol to water may be about 1:1.5 to about 1:15, specifically, it may be about 1:1.8 to about 1:15.

In some embodiments, the reaction in step 2b) is carried out at 0 °C to room temperature.

In some embodiments, the compound of Formula (VII) is the compound shown in Formula (VIIa) or (VIIb),

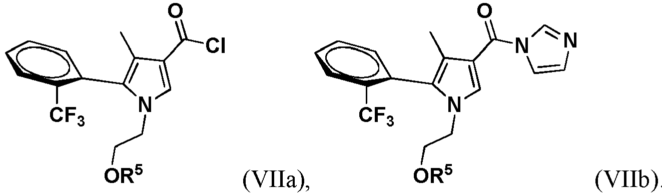

In some embodiments, in step 2b), the compound of Formula (VI) reacts with dichlorosulfoxide or oxalyl chloride to obtain the compound of Formula (VIIa), 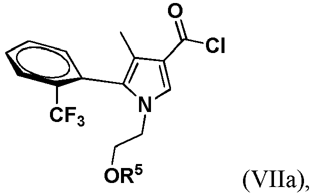 wherein, the reaction is carried out in a suitable solvent.

In other embodiments, the suitable solvent includes, but is not limited to, dichloromethane (DCM) or tetrahydrofuran (THF).

In other embodiments, in step 2b), the compound of Formula (VI) reactes with N,N'-carbonyldiimidazole (CDI) to obtain a compound of Formula (VIIb), 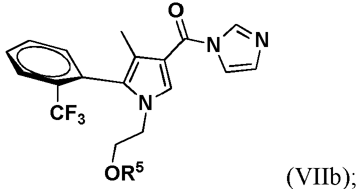 wherein, the reaction is carried out at room temperature in a solvent such as *N,N*-dimethylformamide (DMF) or DMAC.

In some embodiments, the ammonolysis reaction of step 2c) is carried out in the presence of an amination reagent. Preferably, the amination reagent is ammonia or an ammonium salt reagent; and preferably, the ammonia reagent is ammonia, ammonium bromide or NH₄SCN.

In some embodiments, the ammonolysis reaction of step 2c) is further carried out in the presence of base 2, which is potassium carbonate, sodium carbonate or cesium carbonate.

In some embodiments, the amination reagent of step 2c) is ammonium bromide or NH₄SCN, and the ammonolysis reaction of step 2c) is carried out in the presence of base 2, which is potassium carbonate, sodium carbonate or cesium carbonate.

In some embodiments, the temperature of the ammonolysis reaction of step 2c) is 0 °C to room temperature.

In some embodiments, the crude product of the compound of Formula (VIII) obtained by the ammonolysis reaction of step 2c) may be further purified by beating or recrystallization. In other embodiments, the recrystallization comprises: adding a crude product of the compound of formula (VIII) to solvent a, heating and stirring, adding solvent b after the system is dissolved, cooling, stirring to precipitate a solid. In other embodiments, in the recrystallization, the crude product of the compound of Formula (VIII) is added to solvent a, heated to about 80 °C, and stirred until the system is dissolved.

In still other embodiments, the solvent a is isopropyl acetate or *tert*-butyl acetate, and the solvent b is *n*-hexane, *n*-heptane or cyclohexane.

In still other embodiments, the volume ratio of solvent a to solvent b is 1:2 to 4:3.

In still other embodiments, the solvent a is isopropyl acetate and the solvent b is cyclohexane, wherein the volume ratio of cyclohexane to isopropyl acetate is 1:1 to 2:1.

In still other embodiments, the solvent a is *tert*-butyl acetate, the solvent b is cyclohexane, and the volume ratio of cyclohexane to *tert*-butyl acetate is 3:4.

In still other embodiments, the total volume of solvent a and solvent b is 6~7 times the mass of the crude compound of Formula (VIII).

In some embodiments, the reaction of step 2a) is carried out in the presence of base b; and the base b is sodium hydroxide, potassium hydroxide, etc.; preferably, the base b is potassium hydroxide.

In some embodiments, the reaction of step 2a) is carried out in an alcohol solvent. The alcohol solvent is preferably methanol or ethanol.

In another aspect, the present invention provides a method for preparing a compound of Formula (V), 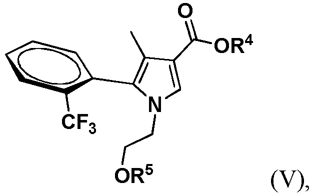 wherein, the method comprises:
1a) a compound of Formula (Ia) reacts with a suitable reagent a to obtain a compound of Formula (III), 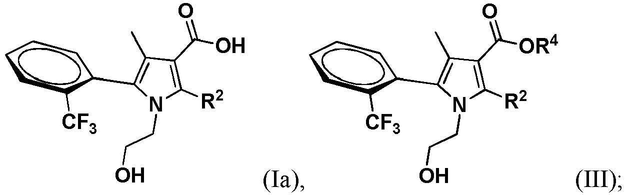
1b) the compound of Formula (III) reacts with a suitable reagent b to obtain a compound of Formula (IV), 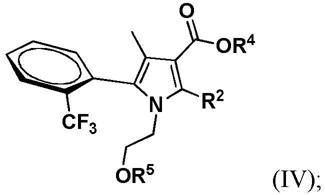
1c) the compound of Formula (IV) reacts to obtain the compound of Formula (V);
wherein, R² is Cl, Br or I; R⁴ is C₁₋₄ alkyl or benzyl, wherein the C₁₋₄ alkyl and benzyl are and optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively;
R⁵ is C₁₋₄ alkyl or benzyl, wherein the C₁₋₄ alkyl and benzyl are optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively.

In some embodiments, R⁴ is methyl, ethyl, *n*-propyl, *i*-propyl, *tert*-butyl or benzyl, wherein the methyl, ethyl, *n*-propyl, *i*-propyl, *tert*-butyl and benzyl are optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively.

In some embodiments, R⁵ is methyl, ethyl, n-propyl, *i*-propyl, *tert*-butyl or benzyl, wherein the methyl, ethyl, *n*-propyl, *i*-propyl, *tert*-butyl and benzyl are optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively.

In some embodiments, the suitable reagent a of step 1a) is C₁₋₄ alkyl iodide, sulfate or optionally substituted benzyl bromide; preferably, the suitable reagent a is methyl iodide, ethyl iodide or dimethyl sulfate. In other embodiments, the optionally substituted benzyl bromide includes unsubstituted benzyl bromide or benzyl bromide optionally substituted with 1, 2, 3 or 4 substitutes independently selected from halo, C₁₋₄ alkyl, C₁₋₄ haloalkyl, C₁₋₄ alkoxy and C₁₋₄ haloalkoxy.

In some embodiments, the step 1a) is carried out in the presence of a base, wherein the base is sodium carbonate, potassium carbonate, cesium carbonate, potassium phosphate, lithium hydroxide or sodium hydroxide; or
the step 1a) is carried out in the presence of H₂SO₄, phosphoric acid, hydrochloric acid or SOCl₂.

In some embodiments, the reaction solvent of step 1a) is acetone, acetonitrile, DMF, DMAc, DMSO, methanol, ethanol, THF, methyl *tert*-butyl ether or any combination thereof.

In some embodiments, the suitable reagent b in step 1b) is benzyl bromide or substituted benzyl bromide, and the substituted benzyl bromide is optionally substituted with 1, 2, 3 or 4 substitutes independently selected from halo, C₁₋₄ alkyl, C₁₋₄ haloalkyl, C₁₋₄ alkoxy and C₁₋₄ haloalkoxy.

In other embodiments, the molar amount of benzyl bromide or substituted benzyl bromide in step 1b) is 1.0 time or more than 1.0 times that of the compound of Formula (III); preferably, the molar amount of benzyl bromide or substituted benzyl bromide is 1.0 to 4.0 times that of the compound of Formula (III); preferably, the molar amount of benzyl bromide or substituted benzyl bromide is 1.0 to 2.5 times that of the compound of Formula (III); preferably, the molar amount of benzyl bromide or substituted benzyl bromide is 1.1 to 1.7 times that of the compound of Formula (III); more preferably, the molar amount of benzyl bromide or substituted benzyl bromide is 1.5 to 1.7 times that of the compound of Formula (III).

In some embodiments, the reaction of step 1b) is carried out in the presence of base a, and the base a is sodium *tert*-pentoxide, sodium *tert*-butoxide, sodium ethoxide, sodium methoxide, sodium hydroxide, tetrabutylamine hydroxide, potassium *tert*-pentoxide, potassium *tert*-butoxide or potassium hydroxide.

In other embodiments, the molar amount of the base 1b) is 1.0 or more times, preferably 1.0 to 4.0 times, and more preferably 1.1 to 2.5 times, that of the compound of Formula (III).

In other embodiments, the reaction of step 1b) is carried out in the presence of sodium *tert*-pentoxide. Optionally, the molar amount of sodium *tert*-pentoxide is 1.0 time or more than 1.0 times that of the compound of Formula (III); preferably, the molar amount of sodium *tert*-pentoxide is 1.0 to 4.0 times that of the compound of Formula (III); preferably, the molar amount of sodium *tert*-pentoxide is 1.0 to 2.5 times that of the compound of Formula (III); preferably, the molar amount of sodium *tert*-pentoxide is 1.1 to 1.7 times that of the compound of Formula (III); more preferably, the molar amount of sodium *tert*-pentoxide is 1.5 to 1.7 times that of the compound of Formula (III).

In some embodiments, the reaction of step 1b) may be further carried out in the presence of sodium iodide. Preferably, the reaction in step 1b) is carried out in the presence of a catalytic amount of sodium iodide, and the catalytic amount includes but is not limited to 0.1, 0.15, or 0.2 equivalents.

In some embodiments, the base 1b) may be added at room or low temperature.

In other embodiments, the sodium *tert*-pentoxide of the present invention is added at room temperature or low temperature; preferably, the sodium *tert*-pentoxide is added under the conditions of 10 °C-30 °C; and more preferably, the sodium *tert*-pentoxide is added under the condition of 10 °C.

In some embodiments, the reaction temperature of step 1b) is from room temperature to 90 °C, preferably from room temperature to 80 °C, or, preferably, from room temperature to 60 °C, or, preferably, from 30 °C to 90 °C, and further preferably, the reaction temperature of step 1b) is 30 °C-90 °C, preferably 30 °C-60 °C, or, more preferably, 35 °C-45 °C.

In some embodiments, the reaction of step 1b) may be carried out in a nitrogen atmosphere, or it also can be carried out without nitrogen protection.

In some embodiments, the reaction solvent of step 1b) is acetonitrile, tetrahydrofuran (THF), *N-*methylpyrrolidone (NMP), dimethyl sulfoxide (DMSO), *N,N-*dimethylacetamide (DMAC) or *N,N-*dimethylformamide (DMF).

In some embodiments, in step 1b), the amount of the reaction solvent is 5-50 mL/g or preferably 10-20 mL/g, based on the mass of the compound shown in Formula (III).

Step 1c) of the present invention is a dehalogenation reaction. In some embodiments, step 1c) is carried out in the presence of a transition metal catalyst, optionally, the transition metal catalyst is palladium carbon, other palladium-containing catalyst such as palladium acetate or the like, nickel, or nickel-containing catalyst such as nickel chloride or the like.

In some embodiments, the step 1c) is carried out in the presence of ammonium formate, sodium formate, sodium hydrogen phosphate, acid, triethylamine or hydrogen.

In some embodiments, the reaction solvent of step 1c) is methanol, ethanol, isopropanol, *tert*-butanol, THF, DMF, ethyl acetate, methyl *tert*-butyl ether, ethylene glycol dimethyl ether, ethylene glycol monomethyl ether or any combination thereof.

In some embodiments, the reaction temperature of step 1c) is 20 °C-80 °C. Optionally, the reaction temperature of step 1c) is 30 °C-70 °C. Preferably, the reaction temperature of the step 1c) is 45 °C-65 °C.

In some embodiments, the method for preparing the compound of Formula (V) also comprises a method for preparing the compound of Formula (Ia), which is described in the present invention.

Optionally, the compound of Formula (V) may be a compound of Formula (Va-1) as shown below, a compound of Formula (III) may be a compound of Formula (IIIa-1) as shown below, and/or a compound of Formula (IV) may be a compound of Formula (IVa-1) as shown below;

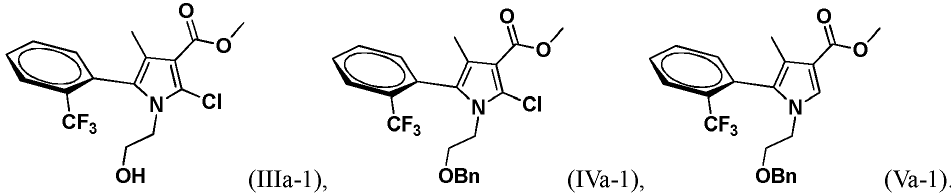

In one aspect, provided herein is a method for preparing the compound of Formula (Ia), 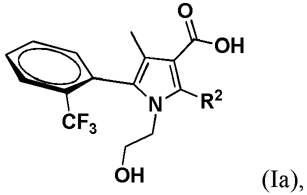 wherein, the method comprises:
a) a compound of Formula (IIa) reacts with an optically active amine to obtain a corresponding salt of the optically active amine of the compound of Formula (Ia), and
b) the resulting salt obtained from step a) reacts to yield the compound of Formula (Ia); 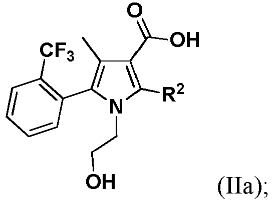 wherein, R² is Cl, Br or I.

In some embodiments, the optically active amine of the present invention is an optically active amine with a quinine skeleton.

In some embodiments, the optically active amine with quinine skeleton of the present invention is quinine, hydroquinine, quinidine, cinchonine or cinchonidine.

In some embodiments, the salt of the optically active amine of the compound described in Formula (Ia) of the present invention is the salt formed by the compound shown in Formula (Ia) and the optically active amine. In other embodiments, the salt of the optically active amine of the compound of Formula (Ia) of the present invention is the quinine salt of the compound of Formula (Ia), the hydroquinine salt of the compound of Formula (Ia), the quinidine salt of the compound of Formula (Ia), the cinchonine salt of the compound of Formula (Ia), or the cinchonidine salt of the compound of Formula (Ia).

In some embodiments, the reaction of step b) of the present invention is a hydrolysis reaction.

In some embodiments, the reaction of step b) of the present invention is carried out under an acidic condition.

In some embodiments, the acidic condition of the present invention is the condition for the existence of hydrochloric acid, sulfuric acid, hydrochloric acid or citric acid.

In some embodiments, the reaction solvent of step a) of the present invention is *N,N*-dimethylformamide (DMF), *N,N-*dimethylacetamide (DMAC), acetonitrile, tetrahydrofuran (THF), ethanol, acetone, isopropyl acetate, ethyl acetate, methyl tert-butyl ether, water, dimethoxyethane or any combination thereof.

In some embodiments, the reaction temperature of step a) of the present invention is from room temperature to 100 °C; preferably, the reaction temperature of step a) is 50 °C-80 °C; preferably, the reaction temperature of step a) is 55 °C-65 °C.

In some embodiments, the preparation method of the compound of Formula (Ia) of the present invention further comprises: spinning the mixed solution obtained after removing the salt of the optically active amine of the compound of Formula (Ia) in step a), adding a suitable solvent, isomerizing by heating to obtain a racemic compound of Formula (IIa), then reacting with the optically active amine to obtain a salt of the optically active amine of the compound of Formula (Ia). In general, the optically active amines described here are the same as those used in step a). Optionally, the suitable solvent includes but is not limited to, DMF, etc.

In other embodiments, optionally, isomerization refers to the racemization of the atropisomer in solution by a mean such as heating to obtain a racemic compound (*i.e.,* the compound of Formula (IIa)). Optionally, a salt of the optically active amine of the compound of Formula (Ia) can be prepared by the racemic compound through the method of step a) of the present invention. Optionally, the compound of Formula (Ia) can be prepared by the salt of the optically active amine of the compound of Formula (Ia) through the method of step b) of the present invention.

The preparation method of a compound of Formula (Ia) shown in the present invention is simple to operate, the yield is high, and the ee/de value of the obtained product is high, and the by-product of R configuration obtained by this method can be recycled by the racemization of the present invention, and the yield of a compound of Formula (Ia) is further improved (up to more than 60%).

In other aspect, the present invention provides a salt formed by a compound shown in Formula (Ia) and an optically active amine, 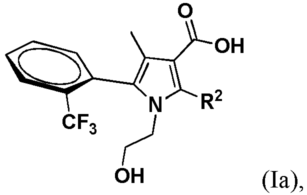 wherein, R² is Cl, Br or I.

In some embodiments, the optically active amine of the present invention is quinine, hydroquinine, quinidine, cinchonine or cinchonidine. Optionally, the optically active amines have the following structures, respectively.

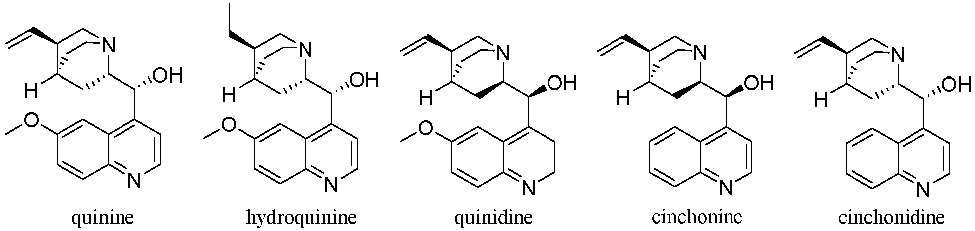

Preferably, the salt formed by the compound shown in Formula (Ia) and an optically active amine is quinine salt of the compound of Formula (Ia), hydroquinine salt of the compound of Formula (Ia), quinidine salt of the compound of Formula (Ia), cinchonine salt of the compound of Formula (Ia), or cinchonidine salt of the compound of Formula (Ia).

In one aspect, the present invention provides a compound of Formula (I), 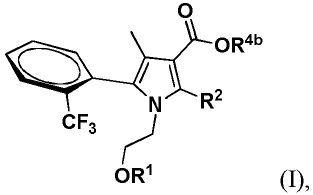
wherein, R^{4b} is H, C₁₋₄ alkyl or optional substituted benzyl;
R¹ is H, C₁₋₄ alkyl or benzyl, and the C₁₋₄ alkyl and benzyl are optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively; and
R² is Cl, Br or I.

Preferably, R¹ of the present invention is H or benzyl.

In some embodiments, the term "optionally substituted benzyl" refers to unsubstituted benzyl, or benzyl substituted with 1, 2, 3 or 4 substitutes independently selected from halo, C₁₋₄ alkyl, C₁₋₄ haloalkyl, C₁₋₄ alkoxy and C₁₋₄ haloalkoxy.

In some embodiments, the compound of Formula (I) of the present invention is a compound shown in Formula (Ia), Formula (Ia-1) or Formula (Ib-1),

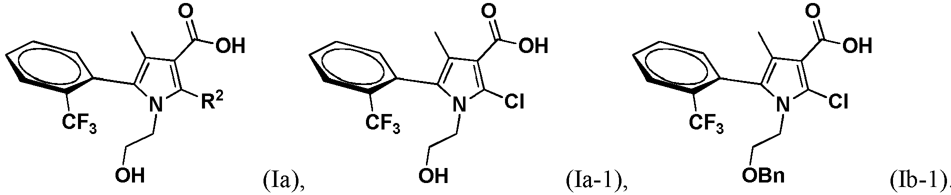

In other aspect, the present invention provides a compound of Formula (II), 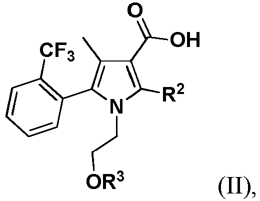 wherein, R² is Cl, Br or I; R³ is H, C₁₋₄ alkyl or benzyl, and the C₁₋₄ alkyl and benzyl are optionally substituted with 1, 2, 3 or 4 substituents independently selected from halo, C₁₋₄ alkyl, C₁₋₄ haloalkyl, C₁₋₄ alkoxy and C₁₋₄ haloalkoxy, respectively.

Preferably, R³ of the present invention is H or benzyl.

In some embodiments, the compound of Formula (II) of the present invention is a compound shown in Formula (II), Formula (IIa-1) or Formula (IIb-2),

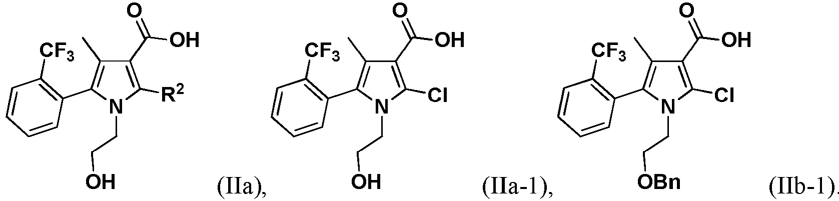

In another aspect, the present invention provides a compound as shown in Formula (IIIa-1), Formula (IVa-1) or Formula (Va-1),

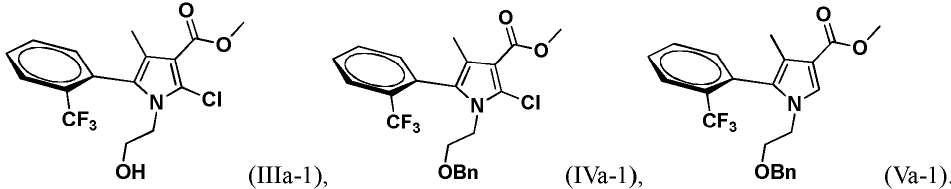

In other aspect, the present invention provides methods for the preparation of compounds of Formulas (Ia-1), (Ib-1), (IIa-1), (IIIa-1), (IVa-1) and/or (Va-1). Optionally, the methods for preparing the compounds of Formulas (Ia-1), (Ib-1), (IIa-1), (IIIa-1), (IVa-1) and/or (Va-1) are described in the present invention.

The compounds of Formulas (I), (Ia), (Ia-1), (Ib-1), (II), (IIa), (IIa-1), (IIb-1), (IIb-2), (III), (IIIa1), (IV), (IVa-1), (V) and/or (Va-1) of the present invention are used to prepare the compound of Formula (A) of the present invention. Preferably, the method for preparing the compound shown in Formula (A) using the aforementioned compounds is described in the present invention.

### DEFINITIONS AND GENERAL TERMINOLOGY

In the present invention, "room temperature" refers to the temperature from about 10°C to about 40°C. In some embodiments, "room temperature" refers to a temperature from about 20 °C to about 30 °C; in other embodiments, "room temperature" refers to 20°C, 22.5°C, 25°C, 27.5°C and the like.

In the context of the present invention, all values disclosed herein are approximate. The value of each number may vary by 1%, 2%, 5%, 7%, 8% or 10%. When a number with an N value is made public, any number within N +/- 1%, N +/- 2%, N +/- 3%, N +/- 5%, N +/- 7%, N +/-8%, or N +/- 10% will be opened clearly, wherein "+/-" means plus or minus. Whenever a lower bound, DL, and an upper limit, DU, of a range of values are disclosed, any values that fall within that range are explicitly disclosed.

After the reaction proceeds to a certain extent in the present invention, such as the raw material is consumed about more than 70%, more than 80%, more than 90%, more than 95%, or completely by monitoring, the reaction mixture is worked up, such as cooled, collected, drawn, filtered, separated, purified or a combination thereof. The reaction can be monitored by conventional method such as thin-layer chromatography (TLC), high performance liquid chromatography (HPLC), gas chromatography (GC), and the like. The reaction mixture can be worked up by conventional method, for example, the crude product can be collected by concentrating the reaction mixture through vacuum evaporation or conventional distillation and is used directly in the next operation; or the crude product can be obtained by filtration of the reaction mixture and is used directly in the next operation; or the crude product can be get by pouring the supernatant liquid of the reaction mixture after standing a while and which is used directly in the next operation; or crude products can be obtained by selecting appropriate organic solvents or their combinations for extraction, distillation, crystallization, column chromatography, rinsing, pulping and other purification steps.

The term "approximately" or "about" in the present invention is used to modify a numerical value with a difference of 10% between the top and the bottom. The term "approximately" or "about" in the present invention is used to modify a numerical value with a difference of 5% between the top and the bottom. The term "approximately" or "about" in the present invention is used to modify a numerical value with a difference of 3% or 2% or 1% between the top and the bottom. It is understandable that the numerical margin of error of an "approximately" or "about" modification is an actual or reasonable margin of error depending on the value it modifies.

The term "optional" or "optionally" refers to that a subsequently described event or circumstance may but need not occur, i.e., the description includes instances where the event or circumstance occurs and instances in which it does not.

In each step of the reaction process described in the present invention, the reaction raw material or other reagent can be added to the reaction system by dropping. The dropping process and each step reaction are carried out under certain temperature conditions, and any temperature suitable for being used in each dropping process or each reaction process is included in the present invention. Additionally, many similar modifications in the art, equivalent replacements, or temperature and temperature scope which are equivalent to those described in the invention, all are deemed to be included in the present invention. The invention gives the optimal temperature or temperature range of each dropping process, and the optimal reaction temperature or reaction temperature range of each reaction.

The expressions "solvent 1", "solvent 2", "solvent a", "solvent b", "baes a", "base b" and the like described in the present invention use Arabic numerals 1, 2, 3...... after "solvent" or "base" or the letters a, b, c....., just to better distinguish the solvents or bases used in the individual steps, the Arabic numerals or letters used have no special meaning. For example, solvent 1, includes any solvent suitable for the reaction of a compound of Formula (II) prepared by the reaction of a compound having Formula (III) with a compound shown in Formula (IV), including but not limited to, toluene, dioxane, dimethyl sulfoxide, *tert*-butanol, *tert*-amyl alcohol, dimethyl ether (DME), cyclopentyl methyl ether (CPME), *N,N*-dimethylacetamide, water or any combination thereof.

The solvent used for the reaction of the invention is not particularly restricted, any solvent is contained in the invention so long as it can dissolve the raw materials to a certain extent and doesn't inhibit the reaction. Additionally, many similar modifications in the art, equivalent replacements, or solvent, solvent composition and the solvent composition with different proportions which are equivalent to those described in the invention, all are deemed to be included in the present invention. The present invention gives the preferred solvent for each reaction step.

The product of each reaction step of the present invention can be purified by recrystallization under suitable conditions. The solvent used for the recrystallization is not particularly restricted, any solvent is contained in the invention so long as it can dissolve the crude product and the recrystallized product can precipitate out under certain conditions. Additionally, many similar modifications in the art, equivalent replacements, or solvent, solvent composition and the solvent composition with different proportions which are equivalent to those described in the invention, all are deemed to be included in the present invention. Wherein the solvent could be alcohol, ethers, alkanes, halohydrocarbons, esters, ketones, aromatic hydrocarbons, acetonitrile, acetic acid, water, DMF, or a combination thereof. Such as water, acetic acid, methanol, ethanol, *n*-propanol, *i*-propanol, *n*-butanol, *i*-butanol, *tert*-butanol, petroleum ether, *n*-pentane, *n*-hexane, *n*-heptane, cyclohexane, DMF, *N,N-*dimethylacetamide, tetrahydrofuran, ethyl ether, isopropyl ether, dioxane, methyl tertiary butyl ether, dimethoxylethane, diethylene glycol dimethyl ether, triethylene glycol dimethyl ether, dichloromethane, 1,2-dichloroethane, chloroform, tetrachloromethane, ethyl acetate, isopropyl acetate, acetone, butanone, benzene, toluene, xylene or a combination thereof.

The amount of water in the solvent described in the present invention is not particularly restricted, that is, the content of water in the solvent does not affect the occurrence of the reaction described in the present invention. So long as the solvent containing a certain amount of water can be used in the reaction disclosed herein, which is deemed to be included in the present invention. The amount of water in the solvent is approximately less than 0.05%, less than 0.1%, less than 0.2%, less than 0.5%, less than 5%, less than 10%, less than 25%, less than 30%, or 0%. In some embodiments, the amount of water in the solvent is within a certain range, which is more conducive to the progress of the reaction; for example, in the step where ethanol is used as the reaction solvent, absolute ethanol is used to facilitate the reaction. In some embodiments, the amount of water in the solvent is outside a certain range, which may affect the progress of the reaction (e.g., affect the yield of the reaction), but does not affect the occurrence of the reaction.

### GENERAL SYNTHETIC PROCEDURES

In the present invention, if the chemical name of the compound doesn't match the corresponding structure, the compound is characterized by the corresponding structure.

In the examples described below, unless otherwise indicated all temperatures are set forth in degrees (°C) Celsius. Unless otherwise indicated, all kinds of materials and reagents are purchased from commodity suppliers and are not further purified when used, and some materials can be prepared according to well-known methods in the art.

1H NMR spectra were recorded by a Bruker Avance 400 MHz spectrometer or Bruker Avance III HD 600 spectrometer, using CDCl₃, *d₆*-DMSO, CD₃OD or *d₆*-acetone (reported in ppm) as solvent, and using TMS (0 ppm) or chloroform (7.25 ppm) as the reference standard. When peak multiplicities are reported, the following abbreviations are used: s (singlet), d (doublet), t (triplet), m (multiplet), br (broadened), dd (doublet of doublets), dt (doublet of triplets), td (triplet of doublets), ddd (doublet of doublet of doublets), ddt (doublet of doublet of triplets), dddd (doublet of doublet of doublet of doublets). Coupling constants, when given, were reported in Hertz (Hz).

Low-resolution mass spectral (MS) data were also determined on an Agilent 6320 series LC-MS spectrometer equipped with G1312A binary pumps and a G1316A TCC (Temperature Control of Column, maintained at 30 °C), a G1329A autosampler and a G1315B DAD detector were used in the analysis. An ESI source was used on the LC-MS spectrometer.

Low-resolution mass spectral (MS) data were also determined on an Agilent 6120 series LC-MS spectrometer equipped with G1311A binary pumps, a G1316A TCC (Temperature Control of Column, maintained at 30 °C), a G1329A autosampler and a G1315D DAD detector were used in the analysis. An ESI source was used on the LC-MS spectrometer.

Both LC-MS spectrometers were equipped with an Agilent Zorbax SB-C18, 2.1 × 30 mm, 5 µm column. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient elution conditions were showed in Table 1:

**Table 1: The gradient condition of the mobile phase in Low-resolution mass spectrum analysis**

| Time (min) | A (CH₃CN, 0.1% HCOOH) | B (H₂O, 0.1% HCOOH) |
|---|---|---|
| 0 - 3 | 5 - 100 | 95 - 0 |
| 3 - 6 | 100 | 0 |
| 6 - 6.1 | 100 - 5 | 0 - 95 |
| 6.1 - 8 | 5 | 95 |

Compound purification was evaluated by Agilent 1260 High Performance Liquid Chromatography (HPLC). Among them, the HPLC is equipped with a G1311B quaternary pump, a G1329B automatic sampler, a G1316A TCC (Temperature Control of Column, maintained at 35 °C), and a G1315D DAD detector. The chromatographic column was Agilent Zorbax Extend C18 (the size was 4.6×150 mm, 5 µm), the flow rate was 1.0 mL/min, the detection wavelength was 250 nm, and the mobile phase and its gradient elution conditions are shown in Table 2-5:

**Table 2: HPLC mobile phases and the gradient elution conditions 1**

| Time (min) | A (acetonitrile) | B (H₂O) |
|---|---|---|
| 0 - 10 | 30-90 | 70-10 |
| 10-25 | 90 | 10 |
| 25-26 | 10 | 90 |
| 26-31 | 90 | 10 |

**Table 3: HPLC mobile phases and the gradient elution conditions 2**

| Time (min) | A (acetonitrile) | B (H₂O) |
|---|---|---|
| 0 - 10 | 10-30 | 90-70 |
| 10-15 | 30-90 | 70-10 |
| 15-20 | 90 | 10 |
| 20-21 | 10 | 90 |
| 21-26 | 10 | 90 |

**Table 4: HPLC mobile phases and the gradient elution conditions 3**

| Time (min) | A (acetonitrile) | B (H₂O) |
|---|---|---|
| 0 - 15 | 10-90 | 90-10 |
| 15-25 | 90 | 10 |
| 25-26 | 10 | 90 |
| 26-31 | 10 | 90 |

**Table 5: HPLC mobile phases and the gradient elution conditions 4**

| Time (min) | A (acetonitrile) | B (0.05% ammonia (pH8.0)) |
|---|---|---|
| 0 - 8 | 10-25 | 90-75 |
| 8-15 | 25-75 | 75-25 |
| 15-20 | 75 | 25 |
| 20-21 | 10 | 90 |
| 21-26 | 10 | 90 |

### EXAMPLES

An embodiment of the present invention discloses methods for preparing pyrrole amide compound as shown in Formula (A) and intermediates thereof. Skilled in the art can learn from this invention to properly improve the process parameters to implement the preparation method. Of particular note is that all similar substitutions and modifications to the skilled person is obvious, and they are deemed to be included in the present in invention. The method of the present invention has been described by a preferred embodiment, related person can clearly realize and apply the techniques disclosed herein by making some changes, appropriate alterations or combinations to the methods without departing from spirit, principles and scope of the present disclosure.

In order to enable those skilled in the art to further understand the invention, it is detailed below through examples.

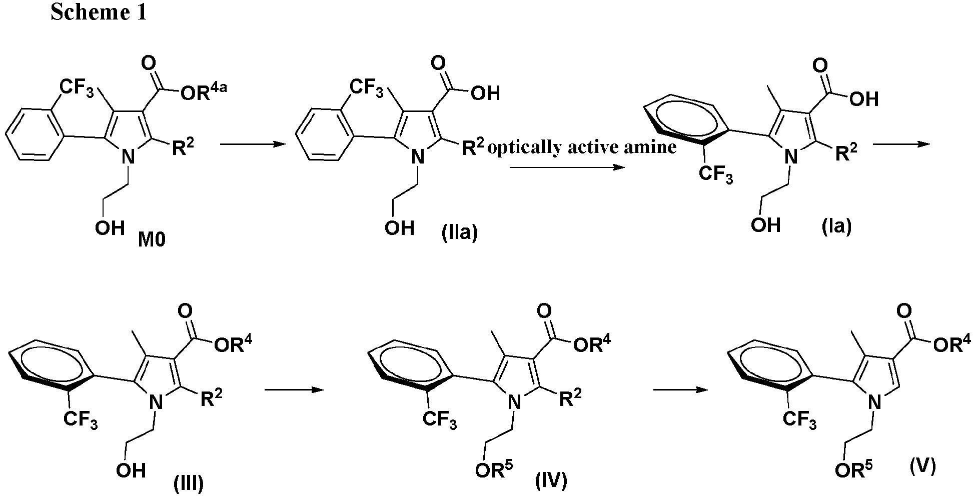

The compound shown in Formula (V) of the present invention can be prepared by the method described in scheme 1, wherein R^{4a} is C₁₋₆ alkyl, preferably methyl or ethyl; and R², R⁴ and R⁵ have the meanings described in the present invention. Compound **M0** is hydrolyzed to obtain the compound of Formula **(IIa).** A salt is formed by the compound of Formula **(IIa)** and an optically active amine (as described in the present invention), and then reacts under a suitable condition (such as an acidic condition, such as under the action of hydrochloric acid) to obtain a compound of Formula **(Ia).** The compound of Formula **(Ia)** reacts with a suitable reagent (*e.g.,* C₁₋₄ alkyl iodide (*e.g.,* methyl iodide or ethyl iodide) or sulfate (*e.g.,* dimethyl sulfate) or optionally substituted benzyl bromide) to obtain a compound of Formula **(III).** The compound of Formula **(III)** reacts with a suitable reagent (such as benzyl bromide, or substituted benzyl bromide as defined in the invention) to obtain a compound of Formula **(IV),** which then undergoes a dehalogenation reaction (*e.g.,* a reaction under the action of a transition metal catalyst (such as a palladium metal catalyst as described in the present invention)) to obtain a compound of Formula (V).

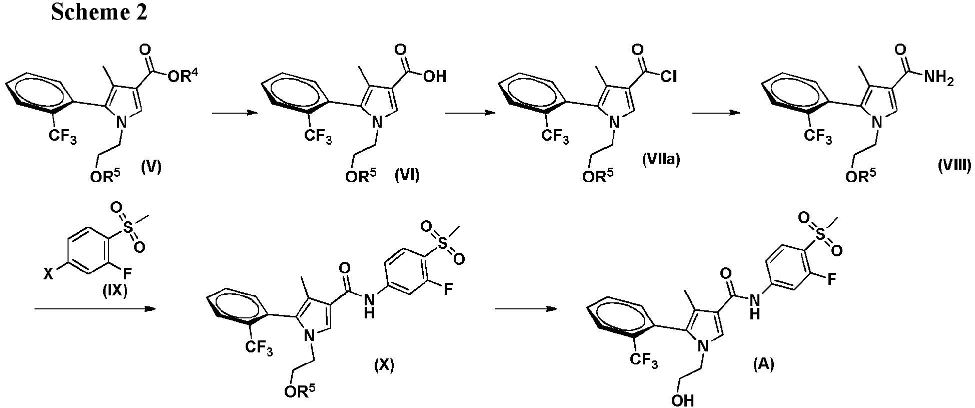

The compound of Formula (A) may be prepared by the method described in scheme 2, wherein X, R⁴ and R⁵ have the definitions described in the invention. The compound of Formula **(V)** is hydrolyzed to obtain the compound of Formula **(VI).** The compound of Formula **(VI)** reacts with a suitable reagent (such as oxalyl chloride, or thionyl chloride, *etc*.) to give the acyl chloride compound shown in Formula **(VIIa).** The compound of Formula **(VIIa)** reacts with an amination reagent (such as ammonia, ammonium bromide, or NH₄SCN) to obtain a compound of Formula **(VIII).** The compound of Formula **(VIII)** and a compound of Formula **(IX)** undergoes a substitution reaction to give a compound of Formula **(X).** Finally, the compound of Formula **(A)** is obtained by removing the protective group from the compound of Formula **(X)** under an acidic condition (*e.g.,* in the presence of hydrochloric acid) or in the presence of an acidic substance (*e.g.,* a Lewis acid such as zinc chloride) and under the action of a metal catalyst (*e.g.,* a palladium metallic catalyst, or palladium-carbon, *etc*.).

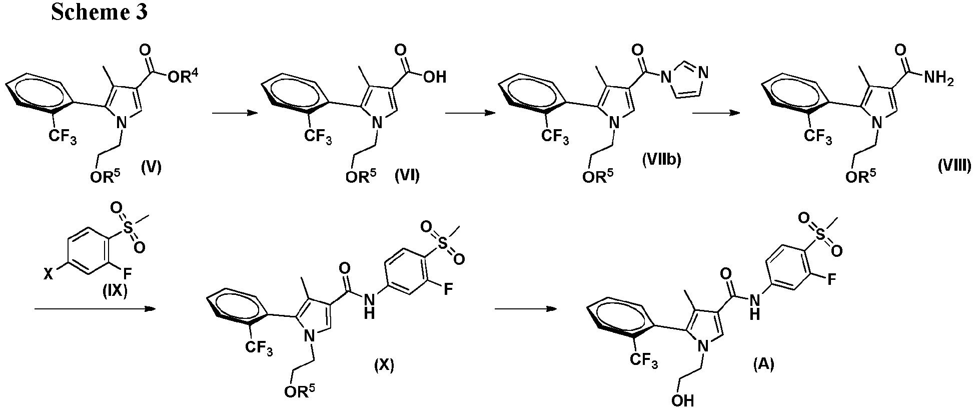

The compound of Formula **(A)** may be prepared by the method described in scheme 2, wherein X, R⁴ and R⁵ have the definitions described in the invention. The compound of Formula **(V)** is hydrolyzed to obtain the compound of Formula **(VI).** The compound of Formula (VI) reacts with *N,N*'-carbonyl diimidazole (CDI) to give the compound shown in Formula **(VIIb).** The compound of Formula **(VIIb)** reacts with an amination reagent (such as ammonia, ammonium bromide, or NH₄SCN) to obtain a compound of Formula **(VIII).** The compound of Formula **(VIII)** and the compound of Formula **(IX)** undergoes a substitution reaction to give a compound of Formula **(X).** Finally, the compound of Formula **(A)** is obtained by removing the protective group under an acidic condition (*e.g.,* in the presence of hydrochloric acid) or in the presence of an acidic substance (*e.g.,* Lewis acid such as zinc chloride) and under the action of a metal catalyst (*e.g.,* a palladium metallic catalyst, or palladium-carbon, *etc*.).

### Example

### Example 1 Preparation of 2-chloro-1-(2-hydroxyethyl)-4-methyl-5-(2-(trifluoromethyl)phenyl)-1H-pyrrole-3-carboxylic acid (intermediate 1)

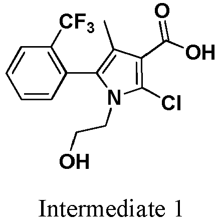

Ethyl 2-chloro-1-(2-hydroxyethyl)-4-methyl-5-(2-(trifluoromethyl)phenyl)-1*H*-pyrrole-3-carboxylate (29.1g, 77.44 mmol) prepared with reference to a well-known method in the art was added to a mixed solvent of methanol (90 mL) and water (30 mL), and then potassium hydroxide (15.34 g, 232.32 mmol) was added and the mixture was heated to reflux for 3 hours. The solvent was evaporated under reduced pressure, water (90 mL) was added to the residue, then the mixture was washed with methyl *tert*-butyl ether (90 mL), the aqueous phase was adjusted to pH=4 with 2 mol/L hydrochloric acid solution, and then extracted with ethyl acetate (150 mL × 2), the organic phases were combined, and washed with water (100 mL) and brine (100 mL), then dried over anhydrous sodium sulfate and filtered with suction, and the filtrate was concentrated under reduced pressure to obtain a light yellow solid of 26.3 g with a yield of 97.67%.

### Example 2 Preparation of (S)-2-chloro-1-(2-hydroxyethyl)-4-methyl-5-(2-(trifluoromethyl) phenyl)-1H-pyrrole-3-carboxylic acid (intermediate 2)

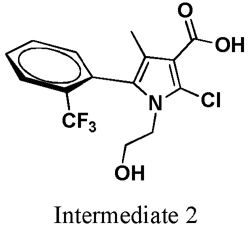

Quinine (7.28 g, 22.43 mmol) was weighed into a reaction flask, *N,N*-dimethylacetamide (6.5 mL), ethyl acetate (65 mL) and water (3.9 mL) were added to the flask and the mixture was heated to 60°C. 2-Chloro-1-(2-hydroxyethyl)-4-methyl-5-(2-(trifluoromethyl)phenyl)-1*H*-pyrrole-3-carboxylic acid (intermediate 1) (13.00 g, 37.39 mmol) was added to a solution of *N,N*-dimethylacetamide (6.5 mL) and ethyl acetate (48 mL), and the mixture was heated and dissolved, and then added dropwise to the aforementioned quinine solution. The heating was turned off, the system was cooled to room temperature, filtered with suction, and the filter cake was rinsed with ethyl acetate (13 mL × 2) and vacuum-dried. The filter cake was added to the mixed solvent of ethyl acetate (80 mL) and water (60 mL), to which was added 2 mol/L hydrochloric acid solution (18.7 mL) under stirring, the aqueous phase was separated after 10 minutes, the organic phase was washed with water (50 mL) and brine (40 mL) in turn and dried over anhydrous sodium sulfate and filtered with suction, and the filtrate was concentrated under reduced pressure to obtain a light yellow solid of 6.35 g, with an *ee* value of 96.04%, a purity of 99.65% and a yield of 48.84%.
MS (ESI, pos. ion) m/z: 314.2 (M+1);
¹H NMR (400 MHz, CDCl3) δ 7.82 (d, *J=* 7.6 Hz, 1H), 7.64 (dt, *J=* 21.3, 7.4 Hz, 2H), 7.43 (d, *J=* 7.3 Hz, 1H), 4.05 (dt, *J=* 10.5, 6.0 Hz, 1H), 3.84 - 3.60 (m, 3H), 2.01 (s, 3H).

### Example 3 Preparation of (S)-methyl 2-chloro-1-(2-hydroxyethyl)-4-methyl-5-(2-(trifluoromethyl)phenyl)-1H-pyrrole-3-carboxylate (intermediate 3)

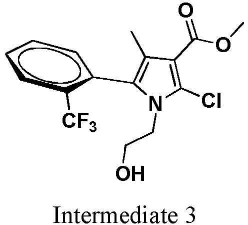

(*S*)-2-chloro-1-(2-hydroxyethyl)-4-methyl-5-(2-(trifluoromethyl)phenyl)-1*H*-pyrro le-3-carboxylic acid (intermediate 2) (6.20 g, 17.83 mmol) and potassium carbonate (5.42 g, 39.23 mmol) were added to acetone (50 mL), then methyl iodide (5.57 g, 39.23 mmol) was added under an ice bath condition. After adding, the mixture was stirred for 14 hours at room temperature. The reaction solution was filtered through a celite pad, eluted with ethyl acetate (30 mL), and the filtrate was concentrated under reduced pressure to obtain 6.40 g of light yellow oil with a yield of 99.23%.

### Example 4 Preparation of (S)-methyl 1-(2-(benzyloxy)ethyl)-2-chloro-4-methyl-5-(2-(trifluoromethyl)phenyl)-1H-pyrrole-3-carboxy late (intermediate 4)

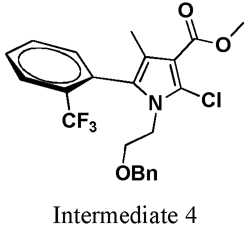

*N,N*-dimethylacetamide (120 mL) was added to (S)-methyl 2-chloro-1-(2-hydroxyethyl)-4-methyl-5-(2-(trifluoromethyl)phenyl)-1*H*-pyrrole-3-carboxylate (intermediate 3) (6.30 g, 17.42 mmol), benzyl bromide (5.06 g, 29.59 mmol) and sodium *tert*-pentoxide (3.26 g, 29.60 mmol) were added to the mixture under an ice bath condition, and the mixture was heated to 40°C for 11 hours. The reaction solution was poured into ice water (120 mL), and the mixture was extracted with methyl *tert*-butyl ether (60 mL × 2). The combined organic phases were washed with water (80 mL × 2) and brine (80 mL), dried over anhydrous sodium sulfate and filtered with suction, and the filtrate was concentrated under reduced pressure to obtain pale yellow oil of 7.85 g with a yield of 99.73%. MS (ESI, pos. ion) m/z: 452.2 (M+1).

### Example 5 Preparation of (S)-methyl 1-(2-(benzyloxy)ethyl)-4-methyl-5-(2-(trifluoromethyl)phenyl)-1H-pyrrole-3-carboxylate (intermediate 5)

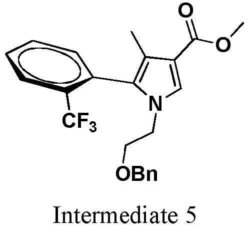

(*S*)-methyl 1-(2-(benzyloxy)ethyl)-2-chloro-4-methyl-5-(2-(trifluoromethyl)phenyl)-1*H*-pyrrole-3-carboxylate (intermediate 4) (7.50 g, 16.60 mmol) was added to methanol (35 mL), then ammonium formate (3.14 g, 49.80 mmol) and 10% palladium carbon (0.75 g) were added, and the mixture was reacted at 55±5 °C for 5 hours under N₂. The reaction solution was filtered through a celite pad, eluted with methanol (10 mL), and the filtrate was concentrated under reduced pressure. Methyl *tert*-butyl ether (40 mL) was added to the residue, and the mixture was washed with water (30 mL) and brine (30 mL) in turn, dried over anhydrous sodium sulfate and filtered with suction, and the filtrate was concentrated under reduced pressure to obtain colorless oil of 6.72 g with a purity of 96.01% and a yield of 96.98%.
MS (ESI, pos. ion) m/z: 418.3 (M+1);
¹H NMR (400 MHz, CDCl₃) δ (ppm) 7.81 - 7.75 (m, 1H), 7.59 - 7.48 (m, 3H), 7.38 - 7.29 (m, 3H), 7.25 - 7.18 (m, 3H), 4.45 (q, *J=* 12.0 Hz, 2H), 3.88 - 3.74 (m, 4H), 3.72 - 3.62 (m, 1H), 3.60 - 3.45 (m, 2H), 2.01 (s, 3H).

Using the intermediate 5 as raw material, a compound of Formula **(A)** can be prepared according to the method of the aforesaid scheme 2 and scheme 3 or the method described in other parts of the present invention or the similar methods disclosed in the prior arts.

Reference throughout this specification to "an embodiment", "some embodiments", "one embodiment", "another example", "an example", "a specific example", or "some examples", means that a particular feature, structure, material, or characteristic described in connection with the embodiment or example is included in at least one embodiment or example of the present disclosure. In this specification, the above terms are not necessarily referring to the same embodiment or example of the present disclosure.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A compound of Formula (I), 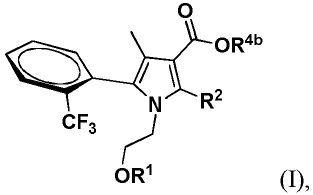
wherein, R^{4b} is H, C₁₋₄ alkyl or an optionally substituted benzyl;
R¹ is H, C₁₋₄ alkyl or benzyl, and the C₁₋₄ alkyl and benzyl are optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively; and
R² is Cl, Br or I.

2. A compound of Formula (II), 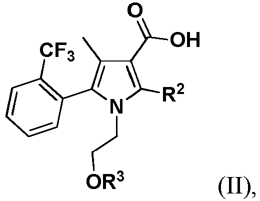
wherein, R² is Cl, Br or I; and
R³ is H or benzyl, and the benzyl is optionally substituted with 1, 2, 3 or 4 substituents independently selected from halo, C₁₋₄ alkyl, C₁₋₄ haloalkyl, C₁₋₄ alkoxy and C₁₋₄ haloalkoxy.

3. A preparation method of a compound of Formula (Ia), 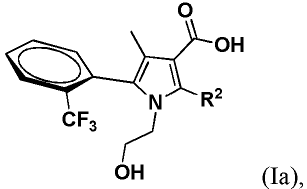 wherein, the method comprises:
a) a compound of Formula (IIa) reacts with an optically active amine to obtain a corresponding salt of the optically active amine of the compound of Formula (Ia), and
b) the salt obtained from step a) reacts to yield the compound of Formula (Ia); 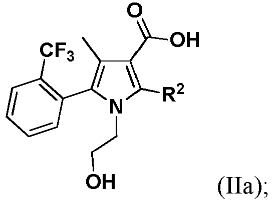 wherein, R² is Cl, Br or I.

4. The preparation method according to claim 3, wherein, the optically active amine is an optically active amine with a quinine skeleton.

5. The preparation method according to claim 4, wherein, the optically active amine with a quinine skeleton is quinine, hydroquinine, quinidine, cinchonine or cinchonidine.

6. The preparation method according to any one of claims 3 to 5, wherein, the reaction of step b) is carried out under an acidic condition.

7. The preparation method according to claim 6, wherein the acidic condition is the condition for the existence of hydrochloric acid, sulfuric acid, hydrochloric acid or citric acid.

8. The preparation method according to any one of claims 3 to 7, wherein, the reaction solvent of step a) is *N,N-*dimethylformamide, *N,N-*dimethylacetamide, acetonitrile, tetrahydrofuran, ethanol, acetone, isopropyl acetate, ethyl acetate, methyl *tert*-butyl ether, water, dimethoxyethane or any combination thereof.

9. The preparation method according to any one of claims 3 to 8, wherein, the reaction temperature of step a) is room temperature to 100 °C; preferably, the reaction temperature of step a) is 50 °C-80 °C; preferably, the reaction temperature of step a) is 55 °C-65 °C.

10. The preparation method according to any one of claims 3 to 9, wherein, the preparation method further comprises: spinning the mixed solution obtained after removing the salt of the optically active amine of the compound of Formula (Ia) in step a), adding a suitable solvent, isomerizing by heating to obtain a racemic compound of Formula (IIa), and then reacting with the optically active amine to obtain a salt of the optically active amine of the compound of Formula (Ia).

11. A salt formed by the compound of Formula (Ia) with an optically active amine, 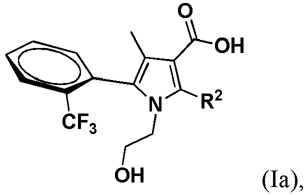 wherein, R² is Cl, Br or I.

12. The salt according to claim 11, wherein, the optically active amine is quinine, hydroquinine, quinidine, cinchonine or cinchonidine.

13. A method for preparing a compound of Formula (V), 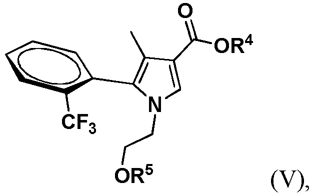 wherein, the method comprises:
1d) a compound of Formula (Ia) reacts with a suitable reagent a to obtain a compound of Formula (III), 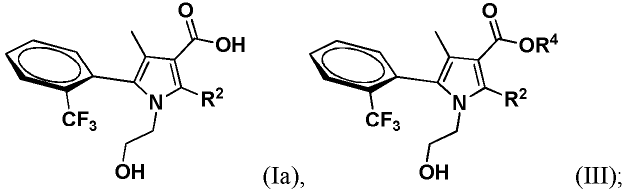
1e) a compound of Formula (III) reacts with a suitable reagent b to obtain a compound of Formula (IV), 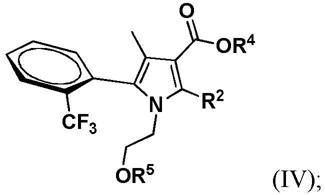
1f) the compound of Formula (IV) reacts to obtain the compound of Formula (V);
wherein, R² is Cl, Br or I;
R⁴ is C₁₋₄ alkyl or benzyl, wherein the C₁₋₄ alkyl and benzyl are optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively;
R⁵ is C₁₋₄ alkyl or benzyl, wherein the C₁₋₄ alkyl and benzyl are optionally substituted with 1, 2, 3 or 4 substitutes independently selected from halo, C₁₋₄ alkyl, C₁₋₄ haloalkyl, C₁₋₄ alkoxy and C₁₋₄ haloalkoxy, respectively.

14. The method according to claim 13, wherein, R⁴ is methyl, ethyl, *i*-propyl, *n*-propyl, *tert*-butyl or benzyl, wherein the benzyl is optionally substituted with 1, 2, 3 or 4 substitutes independently selected from halo, C₁₋₄ alkyl, C₁₋₄ haloalkyl, C₁₋₄ alkoxy and C₁₋₄ haloalkoxy.

15. The method according to claim 13 or 14, wherein, the suitable reagent a of step 1a) is C₁₋₄ alkyl iodide, sulfate ester or optionally substituted benzyl bromide; preferably, the suitable reagent a is methyl iodide, ethyl iodide or dimethyl sulfate.

16. The method according to claims 13-15, wherein, the step 1a) is carried out in the presence of a base, wherein the base is sodium carbonate, potassium carbonate, cesium carbonate, potassium phosphate, lithium hydroxide or sodium hydroxide; or
step 1a) is carried out in the presence of H₂SO₄, phosphoric acid, hydrochloric acid or SOCl₂.

17. The method according to any one of claims 13 to 16, wherein, the reaction solvent of step 1a) is acetone, acetonitrile, DMF, DMAc, DMSO, methanol, ethanol, THF, methyl *tert*-butyl ether or any combination thereof.

18. The method according to any one of claims 13 to 17, wherein, the suitable reagent b of step 1b) is benzyl bromide optionally substituted with 1, 2, 3 or 4 substitutes independently selected from halo, C₁₋₄ alkyl, C₁₋₄ haloalkyl, C₁₋₄ alkoxy and C₁₋₄ haloalkoxy;
optionally, step 1b) is carried out in the presence of sodium tert-pentoxide;
optionally, the reaction solvent of step 1b) is DMAC;
optionally, the reaction temperature of step 1b) is room temperature to 60 °C; preferably, the reaction temperature is 35°C-45°C.

19. The method according to any one of claims 13 to 18, wherein, step 1c) is carried out in the presence of a transition metal catalyst; optionally, the transition metal catalyst is a palladium-carbon catalyst, a palladium acetate catalyst or a nickel catalyst.

20. The method according to any one of claims 13 to 19, wherein, the step 1c) is carried out in the presence of ammonium formate, sodium formate, sodium hydrogen phosphate, acid, triethylamine or hydrogen.

21. The method according to any one of claims 13 to 20, wherein, the reaction solvent of step 1c) is methanol, ethanol, isopropanol, *tert*-butanol, THF, DMF, ethyl acetate, methyl *tert*-butyl ether, ethylene glycol dimethyl ether, ethylene glycol monomethyl ether or any combination thereof.

22. The method according to any one of claims 13 to 21, wherein, the method for preparing the compound of Formula (V) further comprises a method for preparing the compound of Formula (Ia) according to any one of claims 3-10.
